# EUROPEAN PATENT APPLICATION

(11) **EP 2 389 909 A1**
(43) Date of publication of application: **30.11.2011**
(21) Application number: 10733586.1
(22) Date of filing: 25.01.2010
(51) Int. Cl.: A61F 13/15, A61F 13/472

(54) **DEVICE FOR PRODUCING ABSORBENT ARTICLES, METHOD FOR PRODUCING ABSORBENT ARTICLES, AND ABSORBENT ARTICLE**

(30) Priority: 26.01.2009 JP 2009014391
(71) Applicant: Unicharm Corporation, Ehime 799-0111 (JP)
(72) Inventor: KURODA, Kenichiro, Kanonji-shi Kagawa 769-1602 (JP); OKABE, Takayuki, Kanonji-shi Kagawa 769-1602 (JP)
(74) Representative: Eke, Philippa Dianne
(86) International application number: PCT/JP2010/050873
(87) International publication number: WO 2010/084980

(57) **Abstract**

An apparatus 100 of the present invention for manufacturing an absorbent article which includes a liquid-permeable topsheet, a liquid-impermeable backsheet and an absorber provided between the topsheet and the backsheet, is provided with a lower press roller 110B which presses outer edge areas 81 which correspond to outer edges of the extended portions 50 of a continuous body constituted by the plurality of continuous sheets. The lower press roller 110B is provided with a plurality of rear end protrusions 115 which protrude outward in a radial direction of the roller and press rear end inclined areas 81S which correspond to the outside-to-inside inclined areas of the outer edge areas 81. An extending direction LD₂ of the rear end protrusions 115 differ from an extending direction of a rear end outline 87 extending along the shapes of rear end inclined portions 50B in the rear end inclined areas 81S.

## Description

### [Technical Field]

The present invention relates to an apparatus for manufacturing an absorbent article, a method of manufacturing an absorbent article and an absorbent article. The absorbent article includes extended portions which are provided further outside an absorber in a widthwise direction of the absorbent article, extending outward from the absorber in the widthwise direction and constituted by a plurality of sheets.

### [Background Art]

The conventional absorbent articles, such as sanitary napkins and panty liners, have a longitudinally elongated shape extending from foreside to backside of a wearer. An absorbent article typically includes a liquid-permeable topsheet which comes in contact with wearer's skin, a liquid-impermeable backsheet provided further toward a side opposite to the wearer's side than the topsheet, and an absorber provided between the topsheet and the backsheet.

Such an absorbent article includes wing portions (extended portions) which are folded over a crotch portion of panties and are fixed to the panties. The wing portions are provided further outside the absorber in a widthwise direction of the absorbent article and extend outward from the absorber in the widthwise direction.

Each of the wing portions includes a front end inclined portion and a rear end inclined portion. The front end inclined portion inclines rearward as it approaches a widthwise direction outside the absorbent article. The rear end inclined portion is provided nearer the wearer's backside than the front end inclined portion and inclines rearward as it approaches a widthwise direction inside the absorbent article in a direction opposite to the front end inclined portion does (for example, see Patent Document 1).

The wing portions are formed by a plurality of extended sheets, i.e., normally a topsheet and a backsheet. Accordingly embossing is made by a pair of press rollers in the front end inclined portions and the rear end inclined portions of the wing portions. The pair of press rollers presses particular portions of the plurality of sheets so that the sheets are joined together.

Either one of the pair of press rollers includes pressing protrusions which protrude outward in a radial direction of the pressing roller and presses outer edge areas which correspond to outer edges of the wing portions of a continuous body constituted by a plurality of continuous sheets (hereinafter, simply referred to as a continuous body). Specifically the pressing protrusions include a front end protrusion and a rear end protrusion. The front end protrusion presses front end inclined areas which correspond to the front end inclined portions of the outer edge areas. The rear end protrusion presses rear end inclined areas which correspond to the rear end inclined portions of the outer edge areas.

### [Prior Art Document]

### [Patent Document]

### [Patent Document 1]

Japanese Unexamined Patent Application Publication No. 2001-129019 (pages 2 and 3 and Fig. 6)

### [Summary of the Invention]

The related art absorbent articles described above have had the following problems. That is, the front end protrusion presses the front end inclined areas rearward as the front end inclined areas approach the widthwise direction outside the absorbent article while the rear end protrusion presses the rear end inclined areas rearward as the rear end inclined areas approach the widthwise direction inside the absorbent article.

It is therefore a problem that, since the rear end protrusion presses the rear end inclined areas toward the widthwise direction inside the absorbent article, the continuous body is gathered to a central portion and wrinkles are formed in the continuous body This produces defective products.

Accordingly it is an object of the present invention to provide an apparatus for manufacturing an absorbent article, a method of manufacturing an absorbent article and an absorbent article capable of reducing defective products of the absorbent article due to wrinkles formed in a continuous body when a plurality of sheets are joined together by pressing outer edge areas of the continuous body which correspond to extended portions constituted by the plurality of sheets.

In order to achieve the above-described object, the present invention has the following features. The first feature of the present invention is, in summary an apparatus for manufacturing an absorbent article (an apparatus 100 for manufacturing an absorbent article) which includes a liquid-permeable topsheet (a topsheet 10), a liquid-impermeable backsheet (a backsheet 20) and an absorber (an absorber 30) provided between the topsheet and the backsheet, wherein the absorbent article includes extended portions (extended portions 50) which are provided further outside the absorber in a widthwise direction of the absorbent article, extending outward from the absorber in the widthwise direction of the absorber and constituted by a plurality of sheets; the apparatus is provided with a roller (for example, a lower press roller 110B) which presses outer edge areas (outer edge areas 81) which correspond to outer edges of the extended portions of a continuous body constituted by a plurality of sheets; the extended portions includes outside-to-inside inclined areas (rear end inclined areas 51) which include outside-to-inside inclined portions (rear end inclined portions 50B) which incline, from the front to the rear of a rotational direction of the roller, inward from the outside of the continuous body in the widthwise direction; the roller is provided with a plurality of outside-to-inside protrusions (for example, rear end protrusions 115) which protrude outward in a radial direction of the roller and press inclined areas (rear end inclined areas 81S) which correspond to the outside-to-inside inclined areas of the outer edge areas; and extending direction of the outside-to-inside protrusions differ from an extending direction of an outside-to-inside outline (a rear end outline 87) extending along the shapes of the outside-to-inside inclined portions in the inclined areas.

According to the present invention, it is possible to provide an apparatus for manufacturing an absorbent article, a method of manufacturing an absorbent article and an absorbent article capable of reducing defective products of the absorbent article due to wrinkles formed in a continuous body when a plurality of sheets are joined together by pressing outer edge areas of the continuous body which correspond to extended portions constituted by the plurality of sheets.

### [Brief Description of Drawings]

[Fig. 1] Fig. 1 is a perspective view of an absorbent article 1 according to a first embodiment.
[Fig. 2] Fig. 2 is a perspective view of an apparatus 100 for manufacturing the absorbent article according to the first embodiment.
[Fig. 3] Fig. 3 is a perspective view of a lower press roller 110B according to the first embodiment.
[Fig. 4] Fig. 4 is a developed view of the lower press roller 110B according to the first embodiment.
[Fig. 5] Fig. 5 is a diagram illustrating a continuous body 80 which has passed press rollers 110 according to the first embodiment.
[Fig. 6] Fig. 6(a) is a perspective view of an upper cut roller 120A according to the first embodiment and Fig. 6(b) is a developed view of the upper cut roller 120A according to the first embodiment.
[Fig. 7] Fig. 7 is a perspective view of a lower press roller 210 according to a second embodiment.
[Fig. 8] Fig. 8 is a developed view of the lower press roller 210 according to the second embodiment.
[Fig. 9] Fig. 9 is a diagram illustrating a continuous body 80 which has passed press rollers 200 according to the second embodiment.
[Fig. 10] Fig. 10 is a diagram illustrating the continuous body 80 which has passed cut rollers 120 according to the second embodiment.
[Fig. 11] Fig. 11(a) is a perspective view of an upper cut roller 310 according to a third embodiment and Fig. 11(b) is a developed view of the upper cut roller 310 according to the third embodiment.
[Fig. 12] Fig. 12 is a diagram illustrating a continuous body 80 which has passed a cut roller 300 according to the third embodiment.

### [Description of Embodiments]

Hereinafter, an apparatus for manufacturing an absorbent article, a method of manufacturing an absorbent article and an absorbent article according to the present invention will be described with reference to the drawings. Specifically a first embodiment, a second embodiment, a third embodiment and other embodiments will be described.

In the description of the drawings below, the same or similar components are denoted by the same or similar reference numerals. It is to be noted, however, that the drawings are schematic only and not to scale regarding, for example, dimensional ratios.

Accordingly specific dimensions, for example, should be determined in consideration of the description below. It should also be noted that dimensional relationships and ratios may vary among different drawings.

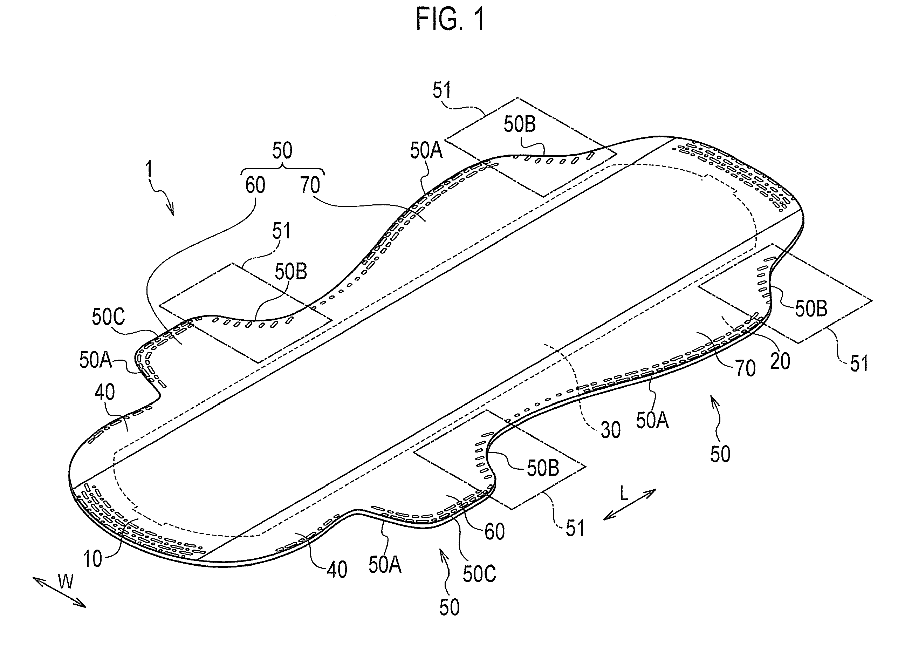

First, a configuration of an absorbent article 1 according to a first embodiment will be described with reference to the drawings. Fig. 1 is a perspective view of the absorbent article 1 according to the first embodiment. The absorbent article 1 according to the first embodiment herein is a sanitary napkin.

As illustrated in Fig. 1, the absorbent article 1 has a longitudinally elongated shape extending from a foreside to a backside of a wearer. The absorbent article 1 includes a liquid-permeable topsheet 10, a liquid-impermeable backsheet 20, an absorber 30 and side sheets 40. The topsheet 10 comes into contact with wearer's skin. The backsheet 20 is provided nearer non-wearer's side than the topsheet 10. The absorber 30 is provided between the topsheet 10 and the backsheet 20. The side sheets 40 are provided on both sides in a widthwise direction W at the side portions in the width direction W of the topsheet 10, which is perpendicular to a longitudinal direction L of the absorbent article 1.

The absorbent article 1 further includes extended portions 50 which are provided further outside the absorbent article 1 in the widthwise direction W than the absorber 30 and extend outward from the absorber 30 in the widthwise direction W

The extended portions 50 are constituted by a plurality of sheets (for example, the backsheet 20 and the side sheets 40). The extended portions 50 are constituted by front wing portions 60 and rear wing portions 70. The front wing portions 60 are folded over a crotch portion (crotch portion) of panties and are fixed to the panties. The rear wing portions 70 are provided further rearward of the wearer than the front wing portions 60 and extend outward in the widthwise direction W of the absorbent article 1 along the shape of the panties.

The extended portions 50 (the front wing portions 60 and the rear wing portions 70) each include a front end inclined portion 50A (an inside-to-outside inclined portion) and a rear end inclined portion 50B (an outside-to-inside inclined portion). The front end inclined portion 50A inclines rearward as it approaches the widthwise direction W outside the absorbent article 1. The rear end inclined portion 50B inclines toward a direction opposite to the front end inclined portion 50A does so that the rear end inclined portion 50B inclines rearward as it approaches the widthwise direction W inside the absorbent article 1.

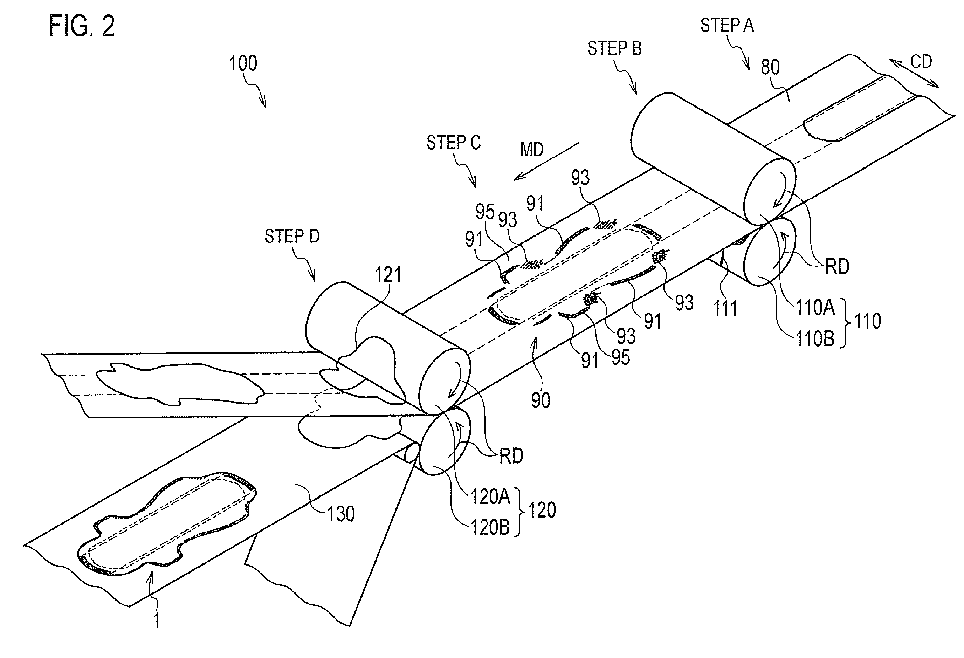

That is, the front end inclined portion 50A inclines, from the front to the rear of a rotational direction RD of the press rollers 110, which will be described later, outward from the inside in a widthwise direction CD of a continuous body constituted by a plurality of continuous sheets (hereinafter, simply referred to as a continuous body 80). Note that the widthwise direction CD of the continuous body 80 is a direction substantially perpendicular to a conveyance direction MD of the continuous body (see Fig. 2).

The rear end inclined portion 50B inclines, from the front to the rear of the rotational direction RD of the press rollers 110, inward from the outside in the widthwise direction CD of the continuous body 80. The rear end inclined portion 50B is provided is included in a rear end inclined area 51 (an outside-to-inside inclined area) which is at the same position as the rear end inclined portion 50B in the widthwise direction CD of the continuous body 80 and is in the widthwise direction CD of the rear end inclined portion 50B.

Here, the front wing portion 60 also includes a connecting portion 50C in addition to the front end inclined portion 50A and the rear end inclined portion 50B. The connecting portion 50C connects the widthwise direction W outside of the front end inclined portion 50A and the widthwise direction W outside of the rear end inclined portion 50B.

Next, a configuration of the apparatus 100 for manufacturing the absorbent article according to the first embodiment will be described with reference to the drawings. Fig. 2 is a perspective view of the apparatus 100 for manufacturing an absorbent article according to the first embodiment.

As illustrated in Fig. 2, the apparatus 100 for manufacturing the absorbent article is provided with a pair of press rollers 110 which presses the continuous body 80 and cut rollers 120 which cut the continuous body 80 which has passed the press rollers 110 into the shape of the absorbent article 1.

The press rollers 110 includes an upper press roller 110A disposed above the continuous body 80 and a lower press roller 110B disposed below the continuous body 80. The upper press roller 110A and the lower press roller 110B rotate about their axes (not illustrated) as the continuous body 80 is conveyed.

The lower press roller 110B includes a plurality of pressing protrusions 111 which protrude outward in a radial direction of the lower press roller 110B. Details of the lower press roller 110B will be described later.

The cut rollers 120 include an upper cut roller 120A disposed above the continuous body 80 and a lower cut roller 120B disposed below the continuous body 80. The upper cut roller 120A and the lower cut roller 120B rotate about their axes (not illustrated) as the continuous body 80 is conveyed.

The upper cut roller 120Aincludes a cutting protrusion 121 which protrudes toward a radial direction outside the upper cut roller 120A. Details of the upper cut roller 120A will be described later.

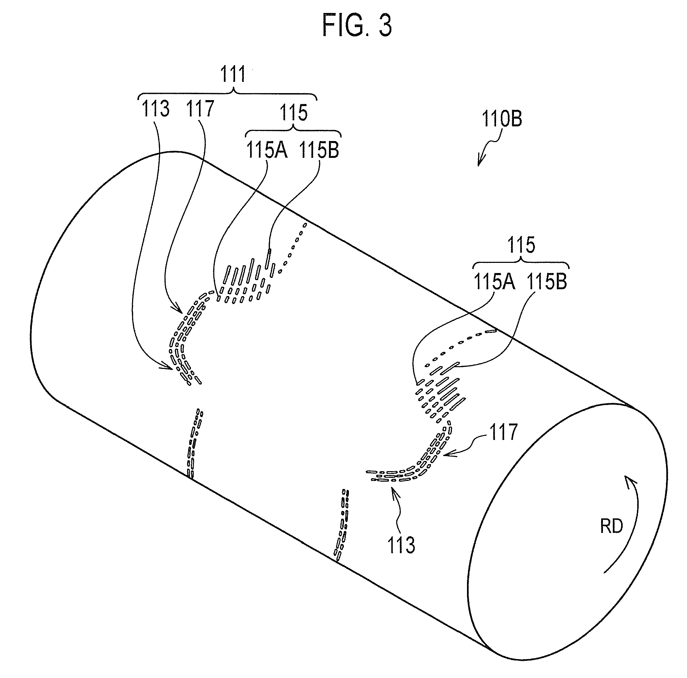

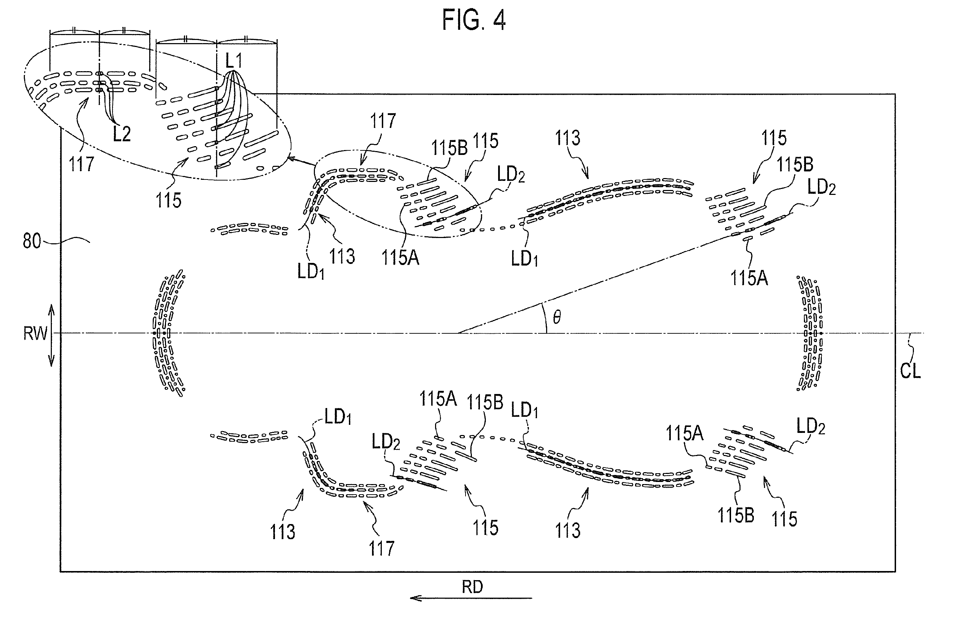

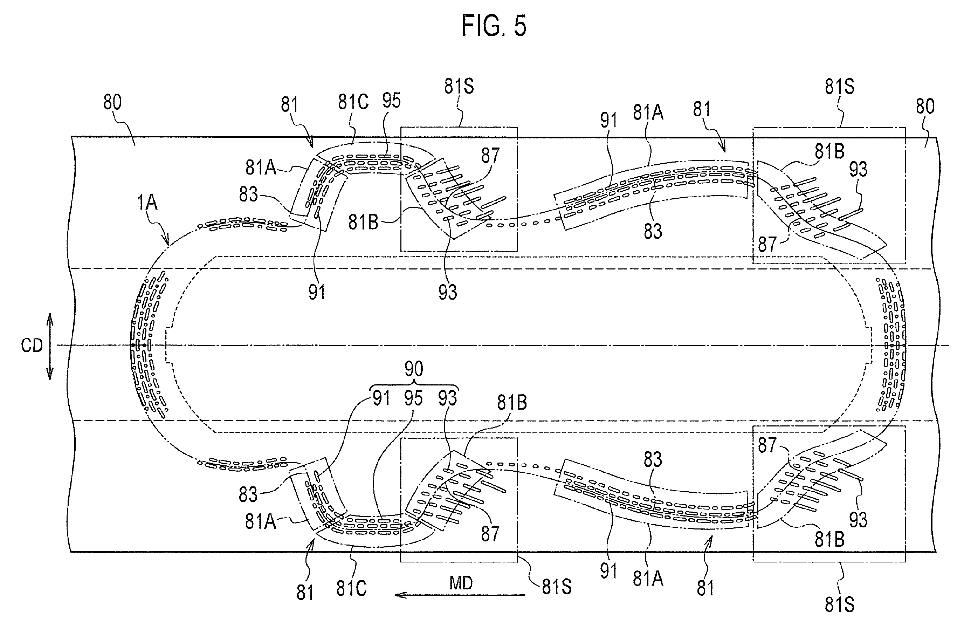

Next, a configuration of the above-described lower press roller 110B will be described with reference to the drawings. Fig. 3 is a perspective view illustrating the lower press roller 110B according to the first embodiment. Fig. 4 is a developed view illustrating the lower press roller 110B according to the first embodiment. Fig. 5 is a diagram illustrating the continuous body 80 which has passed the press rollers 110 according to the first embodiment.

As illustrated in Fig. 3 and Fig. 4, the plurality of pressing protrusions 111 formed in the lower press roller 110B press the continuous body 80 in the shape of the absorbent article 1 illustrated in Fig. 5, i.e., press at a product outline area 1A which corresponds to an outline of the absorbent article 1. That is, the pressing protrusions 111 press the product outline area 1A of the continuous body 80 at outer edge areas 81 which correspond to outer edges of the extended portions 50 (the front wing portions 60 and the rear wing portions 70).

Specifically as illustrated in Fig. 3 to Fig. 5, the pressing protrusions 111 include a plurality of front end protrusions 113 (inside-to-outside protrusions), a plurality of rear end protrusions 115 (outside-to-inside protrusions) and a plurality of intermediate protrusions 117. The front end protrusions 113 press front end inclined areas 81A (inside-to-outside inclined areas) which correspond to the front end inclined portions 50A of the outer edge areas 81. The rear end protrusions 115 press rear end inclined areas 81B (the outside-to-inside inclined areas) which correspond to the rear end inclined portions 50B in the rear end inclined area 81S (inclined areas) which correspond to the rear end inclined areas 51 of the outer edge areas 81. The intermediate protrusions 117 press connecting areas 81C which correspond to the connecting portions 50C of the outer edge areas 81.

The plurality of front end protrusions 113 have longitudinally elongated shapes. The plurality of front end protrusions 113 are arranged at predetermined intervals.

Extending directions LD₁ of the front end protrusions 113 correspond to extending directions of front end outlines 83 (inside-to-outside outlines) extending along the shapes of the front end inclined areas 81A. The extending directions LD₁ of the front end protrusions 113 are directions which cross the centers of the front end protrusions 113 and lie along a longitudinal direction of the front end protrusions 113.

The rear end protrusions 115 have longitudinally elongated shapes which are longer than those of the front end protrusions 113. The rear end protrusions 115 are arranged at predetermined intervals.

The rear end protrusions 115 each include inner-product protrusions 115A and outer-product protrusions 115B. The inner-product protrusions 115A press, after the continuous body 80 is cut by the cut rollers 120, the rear end inclined areas 81B which correspond to the inside of a product configuration of the absorbent article 1. The outer-product protrusions 115B press, after the continuous body 80 is cut by the cut rollers 120, the rear end inclined areas 81B which correspond to the outside of the product configuration of the absorbent article 1.

The inner-product protrusions 115A have longitudinally elongated shapes which are shorter than those of the outer-product protrusions 115B. That is, the outer-product protrusions 115B have longitudinally elongated shapes which are longer than those of the inner-product protrusions 115A.

Extending directions LD₂ of the rear end protrusions 115 (the inner-product protrusions 115A and the outer-product protrusions 115B) differ from the extending directions of the rear end outlines 87 (outside-to-inside outlines) extending along the shapes of the rear end inclined areas 81B. That is, imaginary lines which connect the centers of the plurality of rear end protrusions 115 and extend along the longitudinal directions of the rear end protrusions 115 (namely, the extending directions LD₂) differ from the extending directions of the rear end outlines 87 extending along the shapes of the rear end inclined areas 81B.

The extending directions LD₂ of the rear end protrusions 115 differ from the extending directions LD₁ of the above-described front end protrusions 113. The extending directions LD₂ of the rear end protrusions 115 are directions which cross the centers of the rear end protrusions 115 and extend along the longitudinal directions of the rear end protrusions 115.

The extending directions LD₂ of the rear end protrusions 115 are preferably in a range of -10 degrees and 60 degrees (angle 0) with respect to a widthwise direction center line CL of the continuous body 80. It is especially preferred that the extending directions LD₂ of the rear end protrusions 115 are directed further outward in the widthwise direction CD of the continuous body 80 than the extending directions of the rear end outlines 87. The extending directions LD₂ of the rear end protrusions 115 are all the same.

The plurality of intermediate protrusions 117 have the same shape as those of the plurality of front end protrusions 113. The plurality of intermediate protrusions 117 are arranged at predetermined intervals along a rotational direction RD of the lower press roller 110B.

Here, a total length L1 of the rear end protrusions 115 along a widthwise direction RW of the lower press roller 110B which is perpendicular to the rotational direction RD of the lower press roller 110B is preferably in a range of 30% to 300% with respect to a total length L2 of the intermediate protrusions 117 along the widthwise direction RW of the lower press roller 110B. For example, the total length L1 of the rear end protrusions 115 is 9.0 mm and the total length L2 of the intermediate protrusions 117 is 4.5 mm.

The total length L1 of the rear end protrusions 115 is a value measured at the center of the rotational direction RD of the lower press roller 110B at the rear end protrusions 115. The total length L2 of the intermediate protrusion 117 is a value measured at the center of the rotational direction RD of the lower press roller 110B at the intermediate protrusions 117.

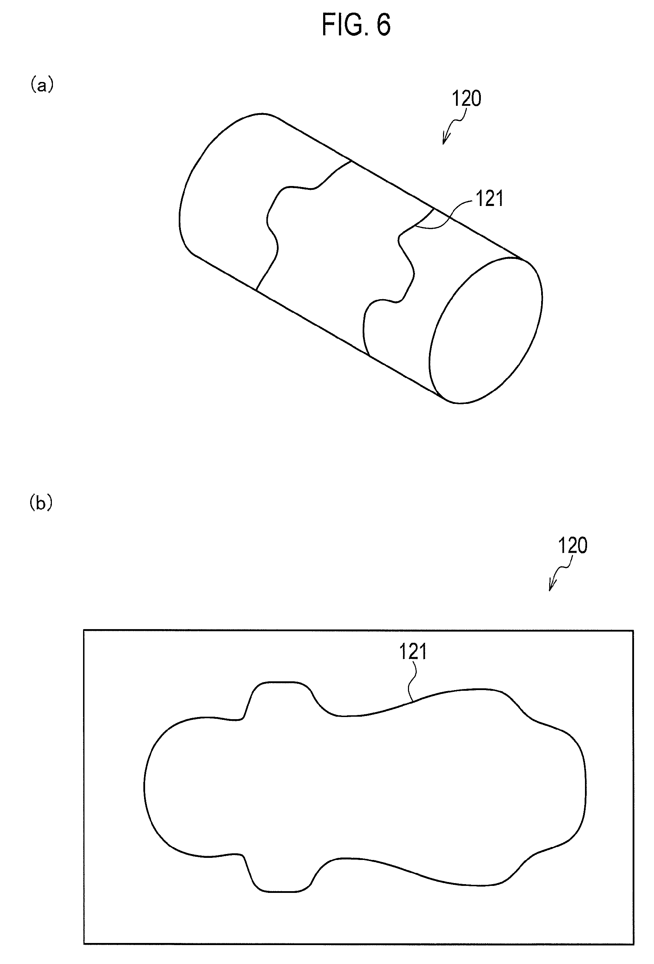

Next, a configuration of the above-described upper cut roller 120A will be described with reference to the drawings. Fig. 6(a) is a perspective view illustrating the upper cut roller 120A according to the first embodiment. Fig. 6(b) is a developed view illustrating the upper cut roller 120A according to the first embodiment.

As illustrated in Fig. 6(a) and Fig. 6(b), the cutting protrusion 121 formed in the upper cut roller 120Apresses the continuous body 80 into the shape of the absorbent article 1, i.e., the product outline area 1A which corresponds to the outline of the absorbent article 1, thereby cutting the product outline area 1A.

Specifically, the cutting protrusion 121 has the same shape as that of the product outline area 1A on a surface of the upper cut roller 120A. Thus the cut rollers 120 produce the absorbent article 1 by cutting the continuous body 80 at the product outline area 1A.

Next, an operation of a method of manufacturing an absorbent article according to the first embodiment will be described briefly with reference to Fig. 2 and Fig. 5.

As illustrated in Fig. 2, the method of manufacturing an absorbent article includes: step A in which the continuous body 80 is conveyed between a pair of press rollers 110; step B in which the continuous body 80 is pressed by the pair of press rollers 110 at the outer edge areas 81; step C in which the continuous body 80 is conveyed to a pair of cut rollers 120; and step D in which the continuous body 80 is cut by the pair of cut rollers 120 at the outer edge areas 81.

In step A, the continuous body 80 is conveyed between the pair of press rollers 110 by a conveyance mechanism (not illustrated), such as a conveyance roller and a belt conveyor. In the first embodiment, the continuous body 80 is conveyed between the pair of press rollers 110 in the order from the front end inclined areas 81A which correspond to the front end inclined portions 50A and then the rear end inclined areas 81B which correspond to the rear end inclined portions 50B.

In step B, the continuous body 80 is pressed by the pair of press rollers 110 at the outer edge areas 81. The pressing protrusions 111 formed in the lower press roller 110B press the continuous body 80 at the outer edge areas 81 to form embossed portions 90 in the outer edge areas 81 (see Fig. 5).

Specifically as illustrated in Fig. 5, the front end protrusions 113 among the pressing protrusions 111 press the front end inclined areas 81A which correspond to the front end inclined portions 50A of the outer edge areas 81 to form front side embossed portions 91 (first pressed portions) in the front end inclined areas 81A.

The rear end protrusions 115 among the pressing protrusions 111 press the rear end inclined areas 81B which correspond to the rear end inclined portions 50B of the outer edge areas 81 to form rear side embossed portions 93 (second pressed portions) in the rear end inclined areas 81B.

The intermediate protrusions 117 among the pressing protrusions 111 press the connecting areas 81C which correspond to the connecting portions 50C of the outer edge areas 81 to form connection embossed portions 95 in the connecting areas 81C.

Here, since the extending directions LD₂ of the rear end protrusions 115 differ from the extending directions LD₁ of the front end protrusions 113, the extending directions of the rear side embossed portions 93 differ from the extending directions of the front side embossed portions 91.

That is, the extending directions of the rear side embossed portions 93 (the first pressed portions) differ from the rear end outlines 87 (the outside-to-inside outlines) extending along the shapes of the rear end inclined portions 50B (the outside-to-inside inclined portions). The extending directions of the front side embossed portions 91 (the second pressed portions) are the same as those of the front end outlines 83 (the inside-to-outside outlines) extending along the shapes of the front end inclined portions 50A (the inside-to-outside inclined portions).

In step C, the continuous body 80 is conveyed between the pair of cut rollers 120 by a conveyance mechanism (not illustrated), such as a conveyance roller and a belt conveyor, as in step A.

In step D, the continuous body 80 which has passed the press rollers 110 is cut by the pair of cut rollers 120 the outer edge areas 81. That is, the cutting protrusion 121 formed in the upper cut roller 120Apresses the product outline area 1A which corresponds to the outline of the absorbent article 1 to cut the continuous body 80 at the product outline area 1A. In this manner, the absorbent article 1 is formed.

As illustrated in Fig. 2, the cut absorbent article 1 is conveyed to the next process by a conveyance mechanism 130 (for example, a belt conveyor). The remaining portion of the continuous body 80 other than the absorbent article 1 is conveyed by for example, a conveyance roller (not illustrated) and collected in a collection device (not illustrated).

In the apparatus 100 for manufacturing an absorbent article and the method of manufacturing an absorbent article according to the first embodiment described above, the extending directions LD₂ of the rear end protrusions 115 differ from the extending directions of the rear end outlines 87 (the outside-to-inside outlines) extending along the shapes of the rear end inclined areas 81B. With this, since gathering of the continuous body 80 to the central portion by the rear end protrusions 115 can be reduced, formation of wrinkles in the continuous body 80 can be prevented as much as possible. It is therefore possible to reduce defective products of the absorbent article 1 due to wrinkles formed in the continuous body 80 when a plurality of sheets are joined together by pressing the outer edge areas 81 of the continuous body 80 which correspond to the extended portions 50 constituted by the plurality of sheets.

In the apparatus 100 for manufacturing an absorbent article and the method of manufacturing an absorbent article according to the first embodiment, the extending directions LD₂ of the rear end protrusions 115 differ from the extending directions LD₁ of the front end protrusions 113. With this, since gathering of the continuous body 80 to the central portion can be reduced to the further extent, formation of wrinkles in the continuous body 80 can be prevented.

In the apparatus 100 for manufacturing an absorbent article and the method of manufacturing an absorbent article according to the first embodiment, the extending directions LD₂ of the rear end protrusions 115 are directed further outward in the widthwise direction CD of the continuous body 80 than the extending directions of the rear end outlines 87. With this, since gathering of the continuous body 80 to the central portion can be reliably reduced, the continuous body 80 can be easily pulled toward the widthwise direction CD outside the continuous body 80 at the rear end protrusions 115 and thus formation of wrinkles in the continuous body 80 can be reliably prevented. Especially since all the extending directions LD₂ of the rear end protrusions 115 are the same, even if the continuous body 80 is about to be gathered to the central portion, the continuous body 80 can be easily pulled toward the widthwise direction CD outside the continuous body 80 at the rear end protrusions 115 which are directed in the same extending directions LD₂.

In the apparatus 100 for manufacturing an absorbent article and the method of manufacturing an absorbent article according to the first embodiment, the extending directions LD₂ of the rear end protrusions are preferably in a range of -10 degrees and 60 degrees (angle 0) with respect to the widthwise direction center line CL of the continuous body 80. If the extending directions LD₂ of the rear end protrusions 115 are smaller than -10 degrees with respect to the widthwise direction center line CL, the continuous body 80 is easily gathered to the central portion and thus it may be unable to prevent formation of wrinkles in the continuous body 80. If the extending directions LD₂ of the rear end protrusions 115 are larger than 60 degrees with respect to the widthwise direction center line CL, the continuous body 80 may be pulled toward the widthwise direction CD outside the continuous body 80 more than needed and thus the continuous body 80 may be stretched.

In the apparatus 100 for manufacturing an absorbent article and the method of manufacturing an absorbent article according to the first embodiment, the total length L1 of the rear end protrusions 115 is preferably in a range of 30% and 300% with respect to the total length L2 of the intermediate protrusions 117. If the total length L1 is smaller than 30% with respect to the total length L2, since pressing force on the continuous body 80 becomes too large locally the continuous body 80 is suddenly pulled toward the widthwise direction CD outside the continuous body 80 and thus wrinkles may be formed. If the total length L1 is larger than 300% with respect to the total length L2, since pressing force on the continuous body 80 becomes too small, the continuous body 80 may be unable to be sufficiently pulled toward the widthwise direction CD outside the continuous body 80 and the continuous body 80 constituted by a plurality of sheets may be unable to be joined.

In the absorbent article 1 according to the first embodiment, the extending directions of the rear side embossed portions 93 differ from the rear end outlines 87 extending along the shapes of the rear end inclined portions 50B and the extending directions of the front side embossed portions 91 is the same as the front end outlines 83 extending along the shapes of the front end inclined portions 50A. With this, since the rear side embossed portions 93 and the front side embossed portions 91 are different in design, the front side and the rear side of the absorbent article 1 can be recognized easily. This is especially effective when no rear wing portions 70 are provided in the absorbent article 1 and the front wing portions 60 are provided at a central portion of the absorbent article in the longitudinal direction L.

If the extending directions of the rear side embossed portions 93 correspond to the longitudinal direction L of the absorbent article 1, the front wing portions 60 are folded over the crotch portion (crotch portion) of panties at the rear side embossed portions 93. With this, the absorbent article 1 can be easily handled.

### [Second Embodiment]

Hereinafter, a configuration of a lower press roller 210 which constitutes press rollers 200 according to a second embodiment according to the present invention will be described with reference to the drawings. Components that are the same as those of the press rollers 110 according to the first embodiment described above will be denoted by the same reference numerals, and the description will be given mainly regarding differences between the first embodiment and the second embodiment.

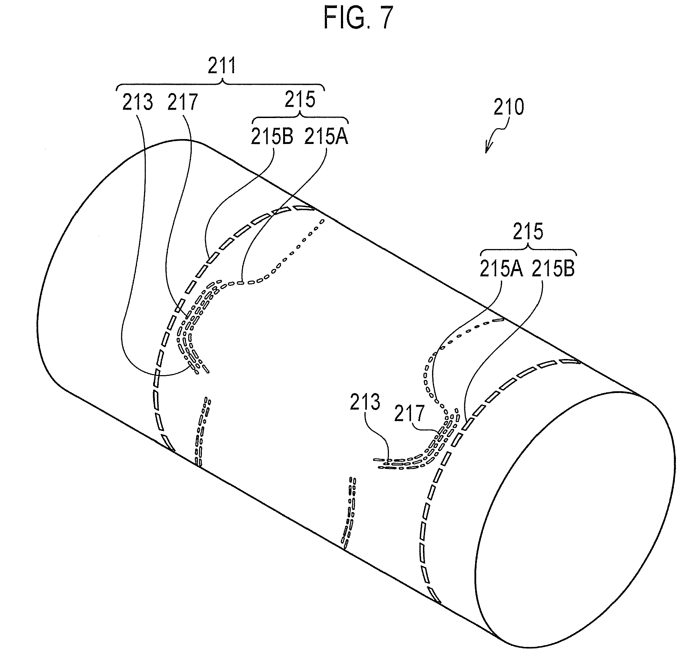

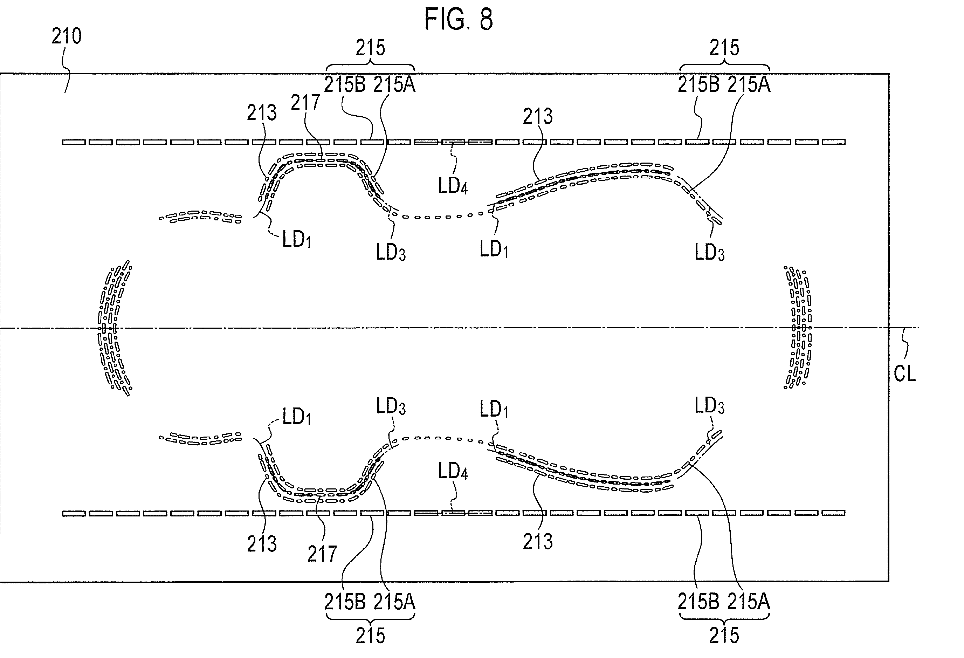

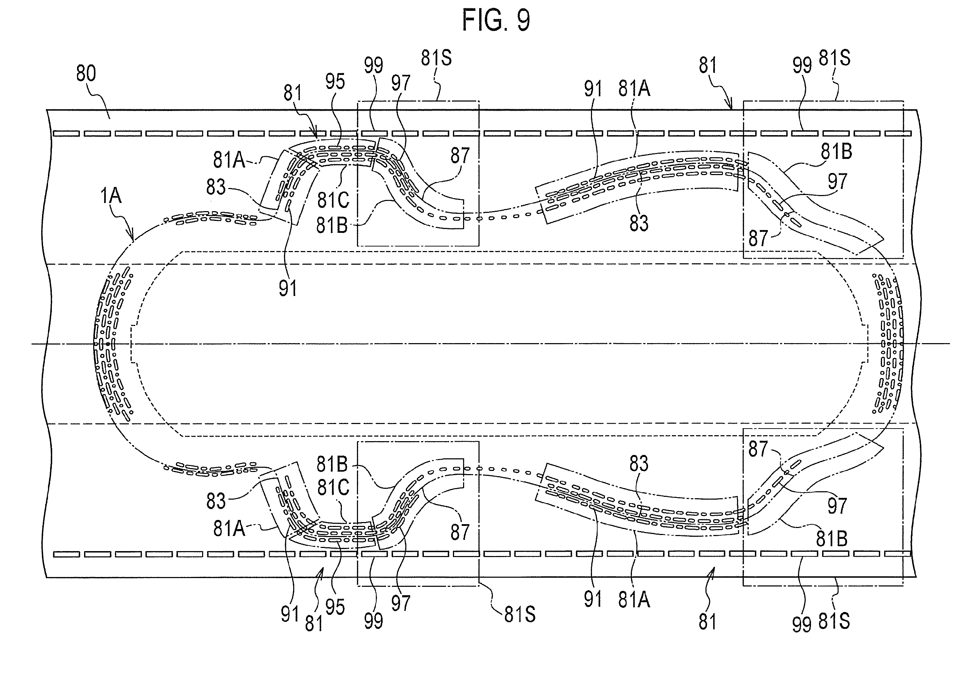

Fig. 7 is a perspective view illustrating the lower press roller 210 according to the second embodiment. Fig. 8 is a developed view illustrating the lower press roller 210 according to the second embodiment. Fig. 9 is a diagram illustrating a continuous body 80 which has passed the press rollers 200 according to the second embodiment.

As illustrated in Fig. 7 to Fig. 9, a plurality of pressing protrusions 211 which protrude outward in a radial direction of the lower press roller 210 are formed in the lower press roller 210. The pressing protrusions 211 press the continuous body 80 in the shape of an absorbent article 1, i.e., press at a product outline area 1A which corresponds to an outline of the absorbent article 1. That is, the pressing protrusions 211 press the product outline area 1A of the continuous body 80 at outer edge areas 81 which correspond to outer edges of the extended portions 50 (front wing portions 60 and rear wing portions 70).

Specifically the pressing protrusions 211 include a plurality of front end protrusions 213, a plurality of rear end protrusions 215 and a plurality of intermediate protrusions 217. The front end protrusions 213 (inside-to-outside protrusions) press front end inclined areas 81A (inside-to-outside inclined areas) which correspond to front end inclined portions 50A of the outer edge areas 81. The plurality of rear end protrusions 215 (outside-to-inside protrusions) press rear end inclined areas 81B (outside-to-inside inclined areas) which correspond to rear end inclined portions 50B of the outer edge areas 81. The intermediate protrusions 217 press connecting areas 81C which correspond to connecting portions 50C of the outer edge areas 81. Configurations of front end protrusions 213 and the intermediate protrusions 217 are the same as those of the front end protrusions 113 and the intermediate protrusion 117 described in the first embodiment.

The rear end protrusions 215 press rear end inclined area 81S which are the same positions as those of is the same position as the rear end inclined portions 50B (the rear end inclined areas 81B) in a widthwise direction CD of the continuous body 80 and which correspond to rear end inclined areas 51 of the outer edge areas 81.

Specifically the rear end protrusions 215 include rear end outline protrusions 215A and auxiliary protrusions 215B. The rear end outline protrusions 215A have similar configurations to those of the front end protrusions 213 and correspond to a rear end outline 87 which extends along the rear end inclined areas 81B in the rear end inclined areas 81S. The auxiliary protrusions 215B have longitudinally elongated shapes which are longer than those of the rear end outline protrusions 215A. Configurations of the rear end outline protrusions 215A are the same as those of the front end protrusions 213.

The auxiliary protrusions 215B press at least a part of the rear end inclined areas 81S which correspond to the rear end inclined areas 51 of the outer edge areas 81. The auxiliary protrusions 215B are provided at the same positions as those of the rear end outline protrusions 215A in the widthwise direction of the continuous body 80 and further outside the rear end inclined areas 81B in the widthwise direction of the continuous body 80. The auxiliary protrusions 215B are provided over the entire circumstance of the lower press roller 210.

Note that it is not necessary to provide the auxiliary protrusions 215B over the entire circumstance of the lower press roller 210; it is only needed that the auxiliary protrusions 215B are provided at least at the same positions as those of the rear end outline protrusions 215A in the widthwise direction of the continuous body 80.

It is not also necessary that the auxiliary protrusions 215B have longitudinally elongated shapes which are longer than those of the rear end outline protrusions 215A; alternatively the auxiliary protrusions 215B may have shapes that are equivalent in length to those of the rear end outline protrusions 215A or shapes that are shorter than those of the rear end outline protrusions 215A.

Next, a configuration of the continuous body 80 which has passed the press rollers 200 will be described with reference to Fig. 9. As illustrated in Fig. 9, the continuous body 80 which has passed the press rollers 200 is pressed at the rear end inclined areas 81B which correspond to the rear end inclined portions 50B of the outer edge areas 81 by the rear end outline protrusions 215A of the pressing protrusions 111. In this manner, rear side embossed portions 97 are formed in the continuous body 80.

Since extending directions LD₃ of the rear end outline protrusions 215A are the same as that of an extending direction of the rear end outline 87 extending along the shapes of the rear end inclined areas 81B, extending directions of the rear side embossed portions 97 are the same as that of an extending direction of the rear end outline 87 extending along the shapes of the rear end inclined areas 81B.

The continuous body 80 which has passed the press rollers 200 is pressed at least a part of the rear end inclined areas 81S which correspond to the rear end inclined areas 51 of the outer edge areas 81 by the auxiliary protrusions 215B of the pressing protrusions 111. In this manner, auxiliary embossed portions 99 are formed in the continuous body 80.

Since extending directions LD₄ of the auxiliary protrusions 215B differ from the extending direction of the rear end outline 87 extending along the shapes of the rear end inclined areas 81B, the extending direction LD₄ of the auxiliary embossed portions 99 differ from the extending direction of the rear end outline 87 extending along the shapes of the rear end inclined areas 81B.

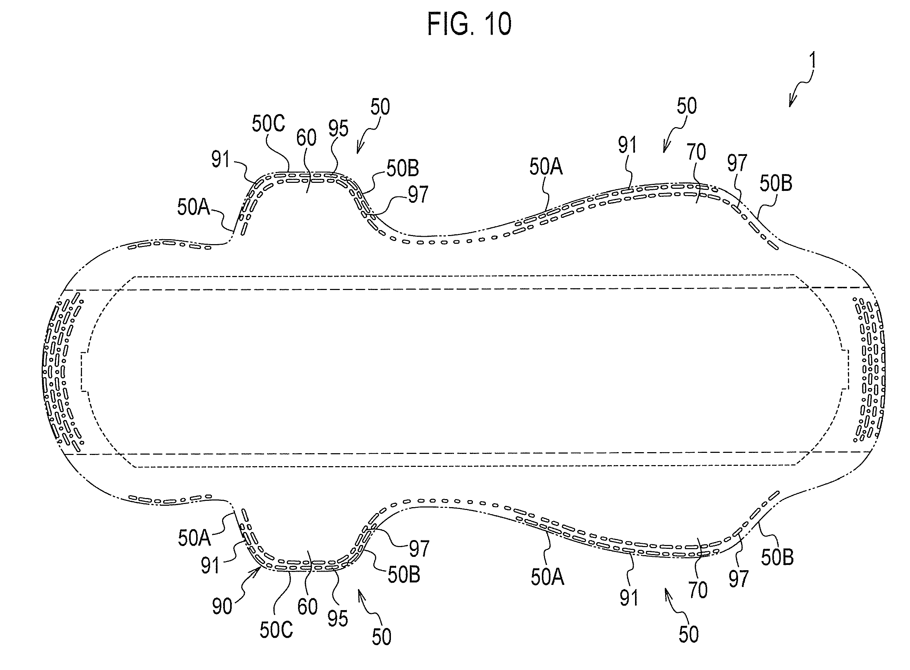

Next, a configuration of the continuous body 80 which has passed the cut rollers 120 will be described with reference to the drawings. Fig. 10 is a diagram illustrating the continuous body 80 which has passed the cut rollers 120 according to the second embodiment.

As illustrated in Fig. 10, the continuous body 80 which has passed the cut rollers 120 is cut at the outer edge areas 81 by a pair of cut rollers 120. In this manner, the cut absorbent article 1 still has the rear side embossed portions 97 but no more has the auxiliary embossed portions 99.

In the second embodiment described above, the auxiliary protrusions 215B of the rear end protrusions 215 are provided at the same positions as those of the rear end outline protrusions 215A in the widthwise direction of the continuous body 80 and further outside the rear end inclined areas 81B in the widthwise direction CD of the continuous body 80. With this, as in the first embodiment, since gathering of the continuous body 80 to the central portion by the auxiliary protrusions 215B can be reduced, formation of wrinkles in the continuous body 80 can be prevented as much as possible. It is therefore possible to reduce defective products of the absorbent article 1 due to wrinkles formed in the continuous body 80 when a plurality of sheets are joined together by pressing the outer edge areas 81 of the continuous body 80 which correspond to the extended portions 50 constituted by the plurality of sheets.

### [Third Embodiment]

Hereinafter, a configuration of an upper cut roller 310 which constitutes cut rollers 300 according to a third embodiment according to the present invention will be described with reference to the drawings. Components that are the same as those of the cut rollers 120 according to the first embodiment described above will be denoted by the same reference numerals, and the description will be given mainly regarding differences between the first embodiment and the third embodiment.

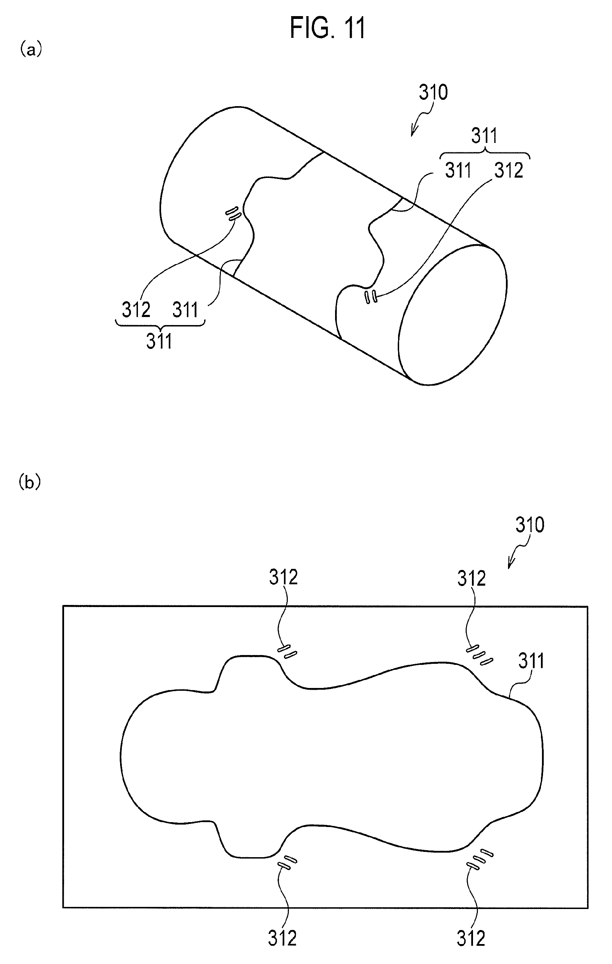

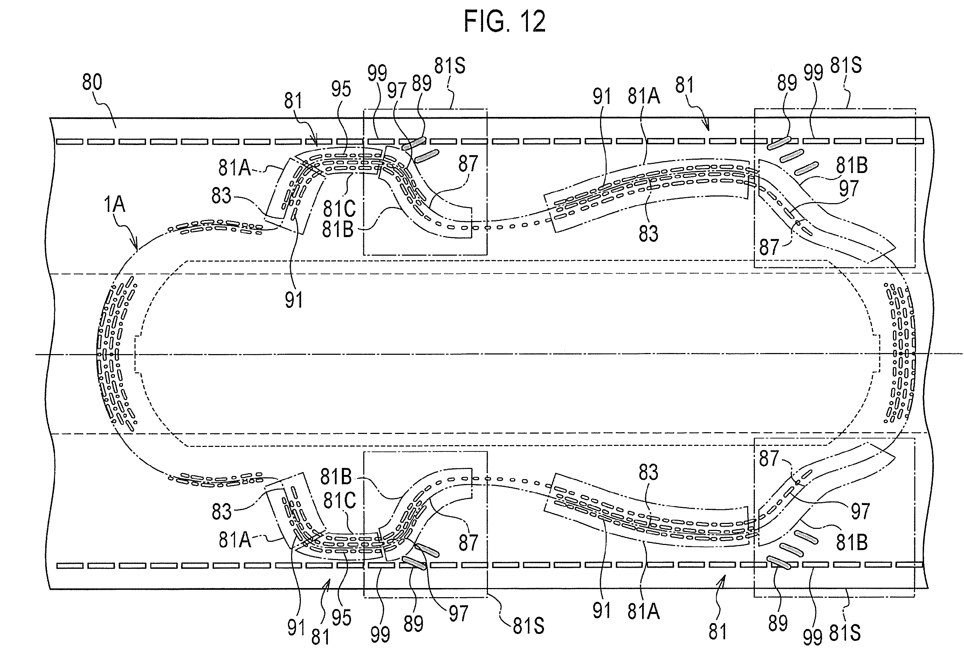

Fig. 11(a) is a perspective view illustrating the upper cut roller 310 according to the third embodiment. Fig. 11(b) is a developed view illustrating the upper cut roller 310 according to the third embodiment. Fig. 12 is a diagram illustrating a continuous body 80 which has passed the cut rollers 300 according to the third embodiment.

As illustrated in Fig. 11 and Fig. 12, the upper cut roller 310 includes cutting protrusions 311 and auxiliary cutting protrusions 312. The cutting protrusions 311 protrude outward in a radial direction of the upper cut roller 310. The auxiliary cutting protrusions 312 protrude outward in the radial direction of the upper cut roller 310 as the cutting protrusions 311 do.

The cutting protrusions 311 press the continuous body 80 in the shape of the absorbent article 1, i.e., press at a product outline area 1A which corresponds to an outline of the absorbent article 1 to cut the continuous body 80 at the product outline area 1A.

The auxiliary cutting protrusions 312 are provided at the same positions as those of the cutting protrusions 311 in the widthwise direction CD of the continuous body 80 and further outside the cutting protrusions 311 in the widthwise direction CD of the continuous body 80. The auxiliary cutting protrusions 312 press at least a part of rear end inclined areas 81S which correspond to rear end inclined areas 51 of outer edge areas 81.

That is, the continuous body 80 which has passed the cut rollers 300 is pressed at least a part of in the rear end inclined areas 81S by the auxiliary cutting protrusions 312. With this, the continuous body 80 is cut into the product outline area 1A, in which process auxiliary embossed portions 89 are formed in the continuous body 80.

In the third embodiment described above, the upper cut roller 310 includes the cutting protrusions 311 and the auxiliary cutting protrusions 312. With this, also in the process of cutting the continuous body 80, since gathering of the continuous body 80 to the central portion by the auxiliary cutting protrusions 312 can be reduced, formation of wrinkles in the continuous body 80 can be prevented as much as possible. It is therefore possible to reliably reduce defective products of the absorbent article 1 due to wrinkles formed in the continuous body 80 when a plurality of sheets are joined together by pressing the outer edge areas 81 of the continuous body 80 which correspond to the extended portions 50 constituted by the plurality of sheets.

### [Other Embodiments]

As described above, while details of the present invention have been disclosed with reference to the embodiments of the present invention, it should not be understood that the discussion and drawings which form a part of the disclosure are restrictive. Various alternatives, examples and operational techniques are obvious for those skilled in the art on the basis of the disclosure.

For example, the embodiments of the present invention can be modified in the following manner. Specifically, the absorbent article 1 has been described as a sanitary napkin, but is not limited to the same the absorbent article 1 may be any articles that have portions extending in the widthwise direction outward the absorber 30. That is, the extended portions 50 have been described to be constituted by the front wing portions 60 and the rear wing portions 70, but are not limited to the same; it is to be noted that the extended portions 50 may be other portions that extend in the widthwise direction outward the absorber 30.

The pressing protrusions 111 have been described to be provided in the lower press roller 110B in the embodiments, but are not limited to the same; the pressing protrusions 111 may alternatively be provided in the upper press roller 110A. The cutting protrusion 121 has been described to be provided in the upper cut roller 120A, but is not limited to the same the cutting protrusion 121 may alternatively be provided in the lower cut roller 120B.

The front end protrusions 113 have been described to have longitudinally elongated shapes in the embodiments, but are not limited to the same; the front end protrusions 113 may alternatively have round shapes, triangular shapes, quadrangular shapes or other shapes.

The outer-product protrusions 115B have been described to have longitudinally elongated shapes which are longer than those of the inner-product protrusions 115A in the embodiments, but are not limited to the same; the outer-product protrusions 115B may have longitudinally elongated shapes that are equivalent in length to those of the inner-product protrusions 115A or longitudinally elongated shapes that are shorter than those of the inner-product protrusions 115A.

It is to be noted that the shape, the configurations, the arrangement and other features of each component which constitutes the apparatus 100 for manufacturing an absorbent article are not particularly limited, and may be selected in accordance with the intended use.

The continuous body 80 has been described to be conveyed between the pair of press rollers 110 in the order from the front end inclined areas 81A which correspond to the front end inclined portions 50A and then the rear end inclined areas 81B which correspond to the rear end inclined portions 50B in the embodiments, but is not limited to the same; the continuous body 80 may alternatively be conveyed between the pair of press rollers 110 in the order from the rear end inclined areas 81B and then the front end inclined areas 81A.

It is to be understood that the present invention encompasses various other embodiments that are not expressly stated herein. Accordingly the technical scope of the present invention shall be solely determined by the matter to define the invention relevant to the appended claims that deem to be appropriate in conjunction with the above description.

Japanese Patent Application No. 2009-014391 (filed January 26, 2009) is incorporated herein in its entirety by reference.

### [Industrial Applicability]

According to the present invention, it is possible to reduce defective products of the absorbent article due to wrinkles formed in the continuous body when a plurality of sheets are joined together by pressing the outer edge areas of the continuous body which correspond to the extended portions constituted by the plurality of sheets. Thus the present invention is useful in an apparatus for manufacturing an absorbent article constituted by a plurality of sheets, such as a sanitary napkin, an incontinence pad and a panty liner.

## Claims

1. An apparatus for manufacturing an absorbent article which includes a liquid-permeable topsheet, a liquid-impermeable backsheet and an absorber provided between the topsheet and the backsheet, wherein:
the absorbent article includes extended portions which are provided further outside the absorber in a widthwise direction of the absorbent article, extending outward from the absorber in the widthwise direction of the absorber and constituted by a plurality of sheets;
the apparatus is provided with a roller which presses outer edge areas which correspond to outer edges of the extended portions of a continuous body constituted by the plurality of continuous sheets;
the extended portions includes outside-to-inside inclined areas which include outside-to-inside inclined portions which incline, from the front to the rear of a rotational direction of the roller,inward from the outside of the continuous body in the widthwise direction;
the roller is provided with a plurality of outside-to-inside protrusions which protrude outward in a radial direction of the roller and press inclined areas which correspond to the outside-to-inside inclined areas of the outer edge areas; and
extending direction of the outside-to-inside protrusions differ from an extending direction of an outside-to-inside outline extending along the shapes of the outside-to-inside inclined portions in the inclined areas.

2. The apparatus for manufacturing an absorbent article according to claim 1, wherein:
the extended portions further include inside-to-outside inclined portions which incline, from the front to the rear of the rotational direction of the roller, outward from the widthwise direction inside the continuous body;
the roller is provided with a plurality of inside-to-outside protrusions which protrude outward in a radial direction of the roller and press inside-to-outside inclined areas which correspond to the inside-to-outside inclined portions of the outer edge areas; and
extending directions of the inside-to-outside protrusions are the same as an extending direction of an inside-to-outside outline extending along the shapes of the inside-to-outside inclined areas and extending directions of the outside-to-inside protrusions differ from the extending directions of the inside-to-outside protrusions.

3. The apparatus for manufacturing an absorbent article according to claim 1 or 2, wherein the extending directions of the outside-to-inside protrusions are directed further outside in the widthwise direction of the continuous body than the extending direction of the outside-to-inside outline.

4. The apparatus for manufacturing an absorbent article according to any one of claims 1 to 3, wherein the plurality of the outside-to-inside protrusions are arranged at predetermined intervals and the extending directions of the outside-to-inside protrusions are all the same.

5. The apparatus for manufacturing an absorbent article according to claim 1 or 2, wherein the outside-to-inside protrusions are provided at the same positions as those of the outside-to-inside inclined portions in the widthwise direction of the continuous body and at positions further outside in the widthwise direction of the continuous body than the outside-to-inside inclined areas which correspond to the outside-to-inside inclined portions of the outer edge areas.

6. A method of manufacturing an absorbent article which includes a liquid-permeable topsheet, a liquid-impermeable backsheet and an absorber provided between the topsheet and the backsheet, wherein:
the absorbent article includes extended portions which are provided further outside the absorber in a widthwise direction of the absorbent article, extending outward from the absorber in the widthwise direction of the absorber and constituted by a plurality of sheets;
the method includes step Ain which a continuous body constituted by a plurality of sheets is conveyed between a pair of rollers which press outer edge areas which correspond to outer edges of the extended portions and step B in which the outer edge areas are pressed by the pair of rollers;
the extended portions includes outside-to-inside inclined areas which include outside-to-inside inclined portions which incline, from the front to the rear of a rotational direction of the roller,inward from the outside of the continuous body in the widthwise direction;
the roller is provided with a plurality of outside-to-inside protrusions which protrude outward in a radial direction of the roller and press inclined areas which correspond to the outside-to-inside inclined areas of the outer edge areas; and
extending direction of the outside-to-inside protrusions differ from an extending direction of an outside-to-inside outline extending along the shapes of the outside-to-inside inclined portions in the inclined areas.

7. An absorbent article which includes a liquid-permeable topsheet, a liquid-impermeable backsheet and an absorber provided between the topsheet and the backsheet, wherein:
the absorbent article further includes extended portions which are provided further outside the absorber in a widthwise direction of the absorbent article, extending outward from the absorber in the widthwise direction of the absorber and constituted by a plurality of sheets;
the extended portions include first pressed portions formed in outside-to-inside inclined portions which incline with respect to a longitudinal direction of the absorbent article by pressing the continuous body constituted by a plurality of sheets and second pressed portions formed in inside-to-outside inclined portions which incline in a direction opposite to the outside-to-inside inclined portions do with respect to the longitudinal direction of the absorbent article by pressing the continuous body;
extending directions of the first pressed portions differ from an outside-to-inside outline extending along the shapes of the outside-to-inside inclined portions; and
extending directions of the second pressed portions are the same as that of an inside-to-outside outline extending along the shapes of the inside-to-outside inclined portions.
